# FASCICULE DE BREVET EUROPEEN

(11) **EP 0 954 345 B1**
(45) Date de publication et mention de la délivrance du brevet: **10.03.2004**
(21) Numéro de dépôt: 97952092.1
(22) Date de dépôt: 17.12.1997
(51) Int. Cl.: A61M 5/32, A61M 5/50, A61M 5/34

(54) **SERINGUE D'INJECTION A PROTECTEUR D'AIGUILLE DEPLACABLE**
INJEKTIONSSPRITZE MIT EINER VERSCHIEBBAREN NADELSCHUTZVORRICHTUNG
INJECTION SYRINGE WITH MOVABLE NEEDLE PROTECTOR

(30) Priorité: 17.12.1996 FR 9615508
(43) Date de publication de la demande: 10.11.1999
(73) Titulaire: SEDAT, 69540 Irigny (Rhône) (FR)
(72) Inventeur: ARNISSOLLE, Yves, F-69230 Saint Genis Laval (FR)
(74) Mandataire: Blot, Philippe Robert Emile
(86) Numéro de dépôt international: PCT/FR1997/002334
(87) Numéro de publication internationale: WO 1998/026824

(56) Documents cités:
- WO-A-96/09849
- FR-A- 2 700 959
- US-A- 5 026 356
- US-A- 5 308 332
- US-A- 5 370 628

## Description

La présente invention concerne une seringue d'injection du type comportant, d'une part un corps de seringue allongé comprenant un tube et une paroi avant munie d'une aiguille d'injection, et d'autre part un piston arrière d'actionnement monté déplaçable dans le tube, laquelle seringue comporte en outre un protecteur mobile de l'extrémité d'injection de l'aiguille, déplaçable entre une position escamotée dans le corps de seringue en retrait de l'extrémité d'injection de l'aiguille, et une position active de protection dans laquelle l'extrémité avant du protecteur se trouve en avant de l'extrémité d'injection de l'aiguille, le protecteur et le corps de seringue comportant des reliefs associés en saillie et en creux de maintien du protecteur dans sa position active de protection.

Une seringue de ce type est décrite par exemple dans le document US-A-5,370,628.

Dans ce document, le protecteur mobile de l'extrémité d'injection de l'aiguille comporte des jambages pénétrant à l'intérieur de la seringue.

Les jambages comportent des moyens d'encliquetage sur le corps de la seringue afin de maintenir le protecteur dans sa position active de protection.

Toutefois, ces moyens d'encliquetage procurent une force de maintien du protecteur relativement faible. Ainsi, une sollicitation axiale exercée sur le protecteur peut provoquer un escamotage de celui-ci à l'intérieur du corps de seringue. Un tel escamotage du protecteur expose à nouveau l'extrémité d'injection de l'aiguille, provoquant ainsi des risques de piqûres accidentelles.

Aussi, dans les seringues connues, la protection de l'extrémité de l'aiguille fournie par le protecteur est peu sûre, le protecteur pouvant relativement facilement être repoussé dans sa position escamotée.

US 5,308,332 décrit une seringue à protecteur d'aiguille extérieure différente du type de seringue objet du brevet.

L'invention a pour but d'apporter une solution à ce problème en limitant en particulier les risques de retour involontaire du protecteur vers sa position escamotée.

A cet effet, l'invention a pour objet une seringue du type précité, caractérisée en ce qu'elle comporte, à l'intérieur du corps de seringue, un organe de retenue positive de l'engagement desdits reliefs associés en saillie et en creux lorsque le pro-tecteur d'aiguille est dans sa position active de protection.

L'invention sera mieux comprise à la lecture de la description qui va suivre, donnée uniquement à titre d'exemple et faite en se référant aux dessins sur lesquels :
- la figure 1 est une vue en coupe longitudinale d'une seringue selon l'invention, avant utilisation ;
- la figure 2 est une vue de détail à plus grande échelle de la zone entourant l'extrémité arrière de l'aiguille de la seringue de la figure 1 ;
- les figures 3 à 6 sont des vues en coupe longitudinale de la seringue de la figure 1 réalisées à différents stades successifs d'utilisation de la seringue,
- la figure 7 est une vue de détail à plus grande échelle de la zone entourant la paroi avant de la seringue après la fin de l'injection, et
- les figures 8 et 9 sont des vues de côté et de détail de moyens prévus entre le corps de seringue et le piston arrière d'actionnement.

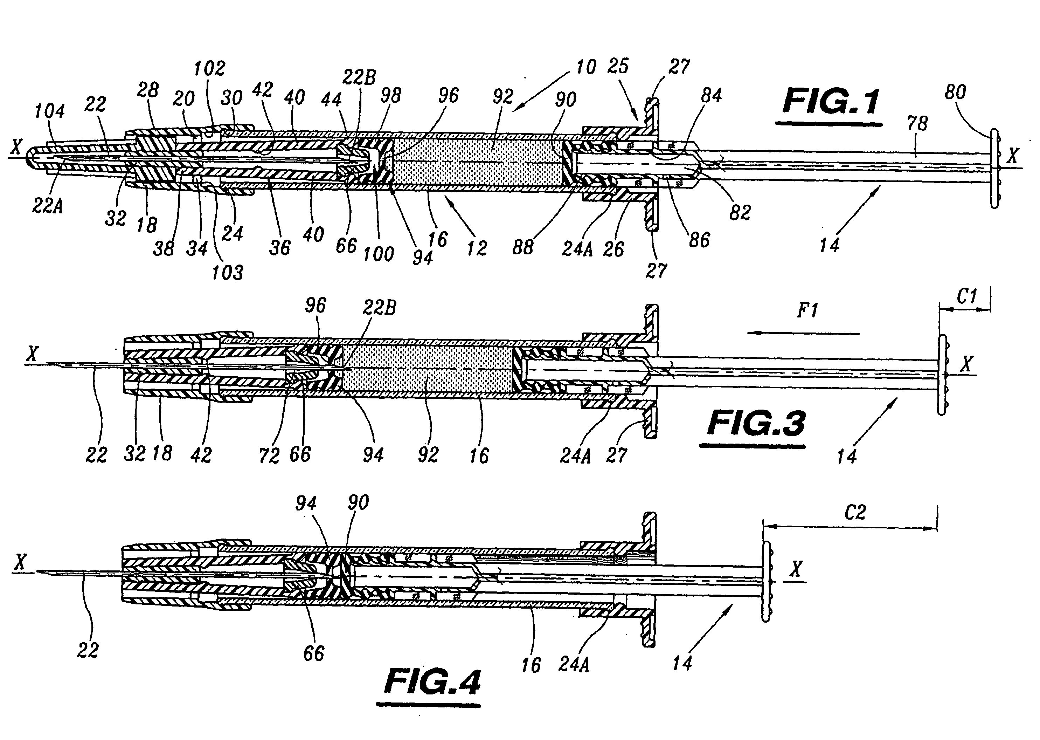

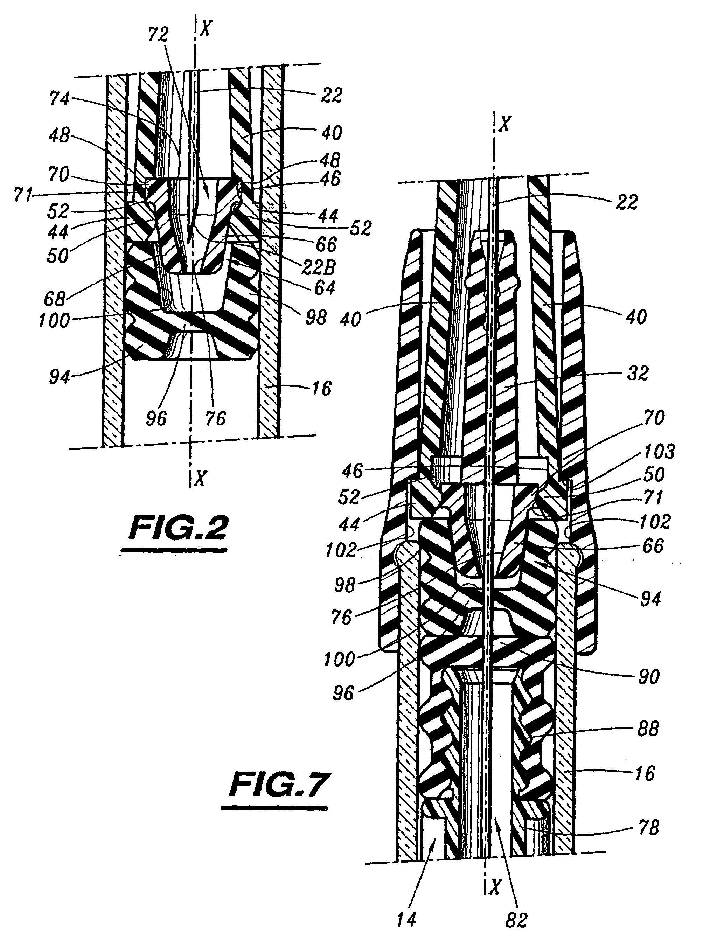

La seringue d'injection 10 représentée sur la figure 1, de forme générale de révolution d'axe X-X, est une seringue à usage unique. Elle est proposée prête à l'emploi et contenant déjà un fluide médical à injecter. Elle comporte essentiellement un corps de seringue 12 allongé et un piston arrière d'actionnement 14 monté déplaçable à l'intérieur du corps 12.

Le corps de seringue 12 est formé d'un tube 16 à l'extrémité avant duquel est fixé un porte-aiguille 18 portant une paroi avant 20 du corps de seringue. Cette paroi avant 20 est munie d'une aiguille d'injection traversante 22. Celle-ci comporte une extrémité avant d'injection 22A faisant saillie par rapport au corps. L'aiguille se prolonge axialement à l'intérieur du corps 12 jusqu'à une extrémité arrière ou proximale 22B. Le tronçon de l'aiguille s'étendant dans le corps de seringue est plus long que le tronçon de l'aiguille faisant saillie en dehors du corps de seringue.

Le tube 16 est réalisé par exemple en verre et a une section circulaire. Son extrémité avant est munie extérieurement d'un bourrelet périphérique 24 de maintien du porte-aiguille 18. De même, à son extrémité arrière, le tube 16 comporte extérieurement un bourrelet périphérique 24A. A cette extrémité arrière est rapporté un organe de préhension 25 facilitant la préhension du corps de seringue entre l'index et le majeur. Celui-ci comporte un manchon 26 encliqueté extérieurement à l'extrémité du tube 16 sur le bourrelet 24A et deux pattes 27 diamétralement opposées pour l'appui des doigts.

Le porte-aiguille 18 est délimité extérieurement par un manchon 28. La paroi avant 20 est venue de matière avec le manchon 28 et s'étend transversalement en un emplacement intermédiaire de celui-ci. Sur la paroi intérieure du manchon 28 est ménagée, légèrement en arrière de la paroi avant 20, une gorge périphérique 30 de réception du bourrelet 24.

La paroi avant 20 présente un plot axial 32 venu de matière, de fixation de l'aiguille d'injection 22. Ce plot est dirigé vers l'extrémité d'injection 22A de l'aiguille et est reçu à l'intérieur de l'espace délimité par le manchon 28.

Trois ouvertures 34 identiques sont ménagées au travers de la paroi avant 20. Elles sont régulièrement réparties angulairement autour du plot 28 sur un même contour circulaire, et présentent une forme arquée. Les figures sont des coupes longitudinales de la seringue effectuées de part et d'autre de l'axe X-X dans la partie médiane de deux des ouvertures 34.

Ces ouvertures 34 assurent le passage et le guidage d'un protecteur d'aiguille 36. Celui-ci comporte à l'avant une bague de protection 38 en matière plastique rigide, dont les diamètres intérieur et extérieur sont adaptés afin que la bague 38 se loge dans l'espace annulaire défini entre le plot 32 et le manchon 18. Cette bague est prolongée par trois jambages 40 identiques élastiquement déformables et espacés angulairement de 120°. Ces jambages 40 présentent en section une légère courbure correspondant à celle de la bague 38 et ont une longueur sensiblement égale à la longueur du tronçon de l'aiguille 22 reçu dans le corps de seringue 12.

Par ailleurs, chaque jambage 40 comporte sur toute sa largeur un premier bombage intérieur 42, disposé légèrement en arrière de la bague 38, et un second bombage extérieur 44 disposé à son extrémité libre.

En outre, comme représenté sur la figure 2, les jambages 40 comportent au voisinage de leurs extrémités libres un chambrage intérieur 46 à fond cylindrique s'étendant sur toute la largeur de chaque jambage. Ce chambrage 46 est délimité sur chaque jambage à l'avant par un épaulement 48 ménagé dans l'épaisseur du jambage et à l'arrière par une saillie 50 ménagée au niveau du bombage 44. Chaque saillie 50 porte une surface de came 52 formée par une surface courbe sensiblement circulaire reliant le sommet arrondi de la saillie 50 au fond du chambrage 46.

Des moyens 64 de guidage axial de l'extrémité arrière 22B de l'aiguille sont retenus entre les extrémités libres des jambages 40. Ces moyens comportent un organe 66 de révolution maintenu entre les jambages 40. Cet organe est formé dans un matériau rigide, notamment une matière plastique rigide. Il comporte extérieurement, à l'arrière, une paroi latérale tronconique 68. Il est bordé extérieurement, à l'avant, par une collerette 70. La surface latérale cylindrique de la collerette 70 est creusée dans son milieu en considérant la direction de l'axe X-X par une gorge périphérique 71 peu profonde. La profondeur de la gorge est en particulier inférieure à la hauteur des bombages 44. Avant utilisation de la seringue (figures 1 et 2), la collerette 70 est reçue dans le chambrage 46 et sa surface latérale s'applique sur le fond cylindrique du chambrage 46. Les bombages 44 des jambages sont alors plaqués contre la surface intérieure du tube 16. Ainsi, l'organe 66 est maintenu centré suivant l'axe X-X par coopération avec la paroi du corps de la seringue.

L'organe 66 comporte un passage axial 72 traversant celui-ci de part en part. Ce passage comporte à l'avant un tronçon cylindrique 74 prolongé par un tronçon 76 de section progressivement décroissante vers l'arrière. Ce tronçon 76 est défini par une surface tronconique dont la section, à son extrémité la plus étroite, est sensiblement égale à celle de l'aiguille 22.

Comme représenté sur la figure 2, avant utilisation de la seringue, l'extrémité arrière 22 de l'aiguille s'étend dans le passage 72, de sorte que l'extrémité de l'aiguille est complètement recouverte par l'organe 66.

Le piston arrière 14 comporte un poussoir allongé 78 ayant une section en croix et présente à son extrémité arrière une pastille 80 d'appui du pouce de l'opérateur. A son extrémité opposée est prévu axialement un logement 82 ouvert sur l'avant, servant à la réception de l'extrémité arrière 22B de l'aiguille en fin d'injection. Ce logement 82, de forme allongée suivant l'axe X-X, a une section circulaire. Il est délimité par une paroi cylindrique 84 munie d'un évent calibré 86.

La paroi 84 présente extérieurement à son extrémité avant un bourrelet hélicoïdal 88 de fixation par emboîtement d'une membrane d'extrémité 90 en forme de cuvette. Cette membrane obture l'ouverture principale avant du logement 82, et constitue une paroi transversale adaptée pour coulisser de manière étanche à l'intérieur du tube 16.

Tel que représenté sur la figure 1, le fluide à injecter 92 est disposé à l'intérieur du tube 16 dans un espace délimité par la membrane 90 du piston arrière et un piston intermédiaire 94. Le piston intermédiaire 94 comporte une paroi transversale perforable 96, entourée par un manchon latéral 98 venu de matière et muni de nervures périphériques extérieures afin d'assurer l'étanchéité au liquide et au gaz entre celui-ci et la paroi latérale intérieure du tube 16. Le piston intermédiaire 94 est appliqué initialement contre l'extrémité du protecteur d'aiguille. La paroi 96 et le manchon 98 délimitent à l'avant une cuvette 100 dans laquelle est partiellement reçue l'extrémité arrière de l'organe 66 qui fait saillie par rapport aux jambages 40.

L'extrémité avant du tube 16 est emmanchée dans le manchon 28 et y est retenue par encliquetage. La gorge périphérique 30 étant prévue légèrement en retrait par rapport à la paroi transversale 20, cette dernière délimite avec l'extrémité avant du tube 16 un canal annulaire 102, disposé immédiatement en arrière de la paroi 20 et dont le fond est formé par le manchon 28. La largeur du canal 102 mesurée suivant l'axe X-X est sensiblement égale au double de la largeur des bombages 44 mesurée suivant ce même axe. A l'avant, le canal 102 est délimité par un épaulement 103 ménagé dans l'épaisseur du manchon 18. La profondeur du creux formé par le canal 102 est sensiblement égale à la hauteur des saillies formées par les bombages 44.

Par ailleurs, un capuchon de protection 104 de l'aiguille 22 est emmanché à l'intérieur du manchon 28 et recouvre l'extrémité d'injection 22A de l'aiguille.

Le montage de la seringue s'effectue de la manière suivante.

Le porte-aiguille 18 est collé sur l'aiguille 22. Le protecteur 36 est monté au travers de la paroi avant par engagement des jambages 40 dans les passages 34. Il est disposé de telle sorte que la bague de protection avant 38 entoure le plot 32 et que les jambages 40 s'étendent le long de la partie arrière de l'aiguille 22. L'organe 66 est ensuite mis en place dans le chambrage 46 par déformation élastique de l'extrémité des jambages 40. L'organe 66 est maintenu en position par l'élasticité des jambages 40 dont les extrémités libres tendent par construction à se rapprocher de l'extrémité 22B de l'aiguille. Le capuchon avant 104 est alors mis en place par emmanchement de celui-ci dans le manchon 28.

Le porte-aiguille 18, ainsi muni du capuchon 104 et de l'organe 66, peut être manipulé sans risque de dégradations des extrémités de l'aiguille, celle-ci étant protégée à ses deux extrémités. En particulier, il peut être distribué sur les chaînes de fabrication dans des bols vibrants.

Parallèlement à l'assemblage du porte-aiguille, le tube 16 est muni de l'organe de préhension 25. Il est rempli du fluide 92 disposé entre le piston intermédiaire 94 et le piston d'actionnement 14. Le porte-aiguille 18 est mis en place par encliquetage à l'extrémité avant du tube 16, comme représenté sur la figure 1.

L'introduction des jambages 40 dans le tube 16 lors du montage du porte-aiguille sur le tube est aisée puisque les jambages 40 associés à l'organe 66 forment un ensemble unitaire et cohérent dont le diamètre extérieur (diamètre mesuré au niveau des bombages 44) correspond exactement au diamètre intérieur du tube 16.

Afin de procéder à l'injection, l'opérateur retire le capuchon 104. De manière classique, l'opérateur exerce alors avec le pouce une poussée sur le piston arrière 14 suivant le sens de la flèche F1, en prenant appui sous les pattes 27 avec l'index et le majeur.

La pression ainsi exercée, transmise par l'intermédiaire du liquide 92 au piston intermédiaire 94, provoque le déplacement de celui-ci suivant une course notée C1 vers l'extrémité proximale 22B de l'aiguille. Cette dernière perfore la paroi 96 du piston intermédiaire au cours du mouvement de celui-ci. Le déplacement du piston intermédiaire 94 s'accompagne du déplacement vers l'avant du protecteur d'aiguille 36.

Lors de l'enfoncement, l'organe 66 est entraîné vers l'avant par le protecteur d'aiguille 36 dans le chambrage 46 duquel il est maintenu. En effet, les jambages 40 sont appliqués suivant les bombages 44 sur la paroi latérale du tube 16 retenant ainsi l'organe 66 dans le chambrage 46.

De plus, lors du mouvement de l'organe 66, la paroi 16 assure, par l'intermédiaire des jambages 40, un centrage et un guidage axial de celui-ci afin de garantir son déplacement rigoureusement suivant l'axe X-X.

Lors du mouvement conjoint du piston intermédiaire 94 et de l'organe 66, l'extrémité arrière 22B de l'aiguille entre d'abord en contact avec la surface tronconique 76. Cette dernière assure, au fur et à mesure du déplacement de l'organe 66, un centrage progressif de l'extrémité 22B de l'aiguille. Elle procure en outre un guidage axial de l'aiguille. Ainsi, lorsque l'extrémité arrière 22B de l'aiguille sort de l'organe 66, le tronçon de l'aiguille disposé à l'intérieur du corps s'étend rigoureusement suivant l'axe X-X. Il y est maintenu par l'extrémité de section réduite du passage 72. L'extrémité arrière 22B de l'aiguille perfore ainsi la paroi 96 en étant toujours retenue latéralement par l'organe 66 disposé légèrement en avant sur l'aiguille.

Dans ces conditions, la perforation du piston intermédiaire 94 s'effectue axialement et dans la partie centrale de celui-ci, garantissant ainsi un déplacement ultérieur aisé du piston 94 sur tout le tronçon intérieur de l'aiguille 22.

Le déplacement du piston intermédiaire 94 empalé sur l'aiguille 22 est stoppé à l'issue de la course C1 lorsque les bombages 42 viennent en butée sur le plot 32, comme cela est représenté sur la figure 3. A cet effet, les bombages 42 sont dimensionnés de telle sorte que le protecteur 36 soit maintenu dans sa position rétractée de la figure 3 dans laquelle la bague 38 s'étend dans le manchon 18 malgré la pression exercée par le piston intermédiaire.

La course C1 correspond à la purge de la seringue. En effet, le déplacement du piston arrière 14 alors que le piston intermédiaire 94 est perforé par l'aiguille assure l'évacuation de l'air contenu dans l'aiguille et l'écoulement d'une faible quantité du fluide 92 au travers de celle-ci.

Après cette purge, l'extrémité d'injection 22A de l'aiguille est introduite dans les tissus du patient.

Le fluide 92 est alors injecté au travers de l'aiguille 22 sous l'effet de la poussée du piston arrière 14 enfoncé suivant une course C2 jusqu'à la position représentée sur la figure 4. Dans cette position, l'essentiel du fluide 92 est injecté et la membrane 90 vient au contact de la surface arrière du piston intermédiaire 94.

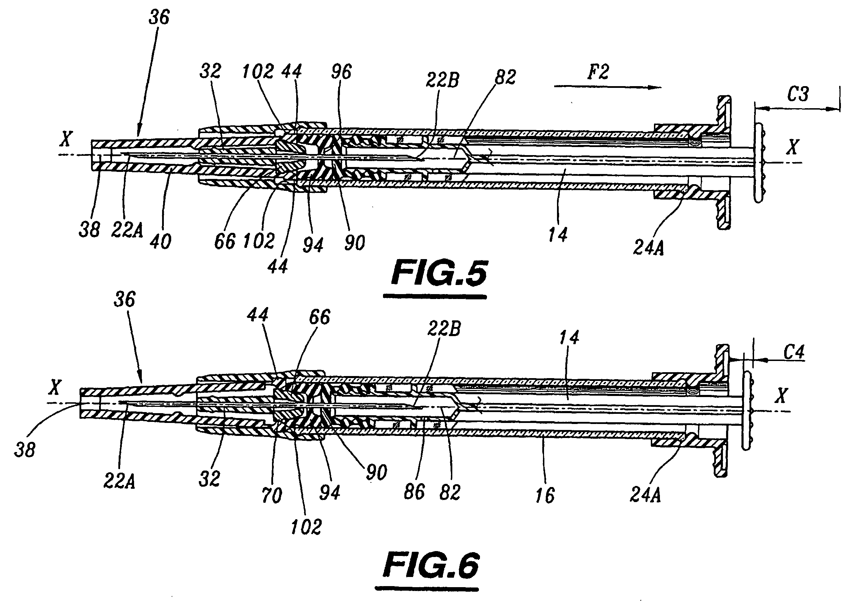

La pression continue exercée par l'opérateur sur le piston arrière 14 provoque ensuite le dégagement des bombages 42 par déformation élastique des jambages 40. Il s'ensuit le déplacement vers l'avant du protecteur 36. Lorsque la bague 38 entre en contact avec la peau du patient, la course du protecteur est stoppée et la poursuite du rapprochement suivant une course C3 du pouce appuyé sur la pastille 80 et des autres doigts maintenus contre les pattes 27 provoque la remontée du corps de seringue 12 dans le sens de la flèche F2 (figure 5).

On comprend que la remontée du corps 12 provoque l'extraction de l'aiguille 22 d'injection du corps du patient.

Il est également possible par simple traction sur le corps de seringue d'extraire l'aiguille des tissus du patient alors que la seringue est dans la position représentée sur la figure 4. L'enfoncement du piston arrière 14 dans le corps de seringue est alors poursuivi avec la seringue dégagée de tout contact avec le patient.

Par ailleurs, l'extrémité arrière 22B de l'aiguille transperce la membrane 90 et est reçue dans le logement 82. Du fait de la présence de l'organe 66, la perforation de la membrane 90 s'effectue rigoureusement suivant l'axe X-X.

Dans la position représentée sur la figure 5, les bombages 44 portés extérieurement par l'extrémité libre des jambages 40 sont disposés en regard du canal annulaire 102. De plus, dans cette position, l'organe 66 s'appuie en butée suivant sa face avant sur le plot 32 et la face avant 20. Il est ainsi immobilisé axialement.

Lors de l'ultime phase d'enfoncement du piston 14 suivant une course C4, le protecteur d'aiguille 36 est amené à se déplacer vers l'avant sous l'action de la poussée du piston arrière 14 transmise par le piston intermédiaire 94 qui est toujours en contact avec l'extrémité libre des jambages 44. A cet effet, l'extrémité arrière de l'organe 66 qui fait saillie par rapport aux jambages 40 s'escamote dans la cuvette 100 permettant ainsi le coulissement du piston intermédiaire 94 malgré l'immobilisation de l'organe 66.

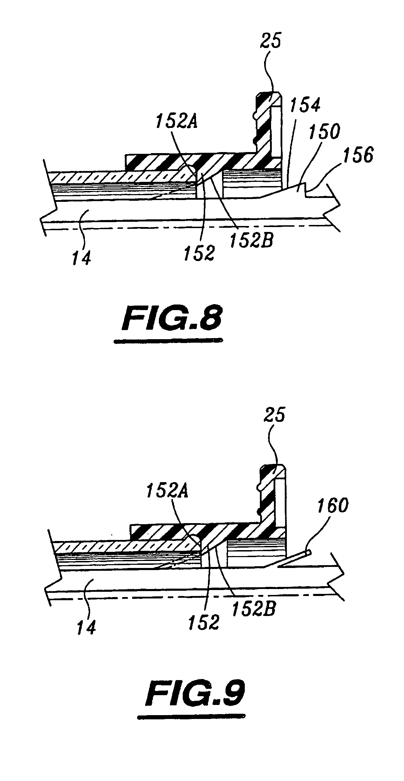

Alors que l'organe 66 est immobilisé, les surfaces de cames 52, ménagées à l'arrière du chambrage 46 coopèrent avec la collerette 70 formant contre-came pour provoquer l'écartement des extrémités libres des jambages. Suite à cet écartement, les bombages 44 sont reçus dans le canal annulaire 102, comme cela est représenté à plus grande échelle sur la figure 7.

Dans cette position, les saillies 50 bordant à l'arrière le chambrage 46 reposent sur la surface latérale de la collerette 70 assurant ainsi une retenue positive des bombages 44 à l'intérieur du canal annulaire 102 et un blocage positif du protecteur d'aiguille 36.

De plus, les saillies 50 sont reçues dans la gorge périphérique 71 de la collerette assurant ainsi une solidarisation axiale de l'organe 66 et du protecteur d'aiguille 36. Ainsi, même si le protecteur d'aiguille est amené à se déplacer légèrement axialement (suivant la longueur du canal annulaire 102), l'organe 66 est entraîné et reste en regard des bombages 44, ce qui permet la retenue positive permanente des bombages 44 dans le canal 102. Ainsi, les extrémités des jambages 40 ne peuvent pas être libérées, ce qui garantit le verrouillage du protecteur d'aiguille 36 dans sa position active de protection.

Sur les figures 6 et 7, le protecteur 36 est en position active de protection et s'étend autour de l'extrémité d'injection 22A de l'aiguille. La face avant de la bague de protection 38 est ainsi située légèrement en avant de l'extrémité 22A de l'aiguille d'injection, interdisant tout contact de celle-ci par un élément extérieur et évitant ainsi tout risque de piqûre contaminante pour l'opérateur.

On comprend que les bombages 44 engagés dans le canal 102 entre l'épaulement 103 et l'extrémité du tube 16 maintiennent solidement le protecteur 36 en position de protection, interdisant ainsi tout escamotage accidentel. Ce maintien est en effet garanti par la présence de l'organe 66 dont la collerette 70 retient écartées les extrémités des jambages 40.

La membrane 90 ayant été perforée par l'extrémité arrière 22B de l'aiguille, la seringue est rendue impropre à une nouvelle utilisation. En effet, cette perforation interdit l'effet d'aspiration obtenu normalement à l'intérieur du corps lors du recul du piston arrière, du fait de la présence de l'évent 86. De même, le piston arrière perforé ne permet pas l'expulsion par l'aiguille d'injection 22 d'un éventuel liquide réintroduit dans le corps de seringue.

On comprend que dans une seringue telle que décrite ici, l'organe 66 garantit une perforation correcte du piston intermédiaire 94. En effet, l'extrémité 22B de l'aiguille guidée par la surface tronconique 76 pénètre au centre du piston 96 et suivant l'axe X-X de la seringue.

Dans une seringue dépourvue de protecteur d'aiguille, l'organe de guidage 66 prend appui suivant sa paroi latérale directement sur la surface intérieure du tube 16 le long duquel il coulisse lors de l'actionnement de la seringue.

Dans l'exemple décrit, l'organe 66 est initialement disposé autour de l'extrémité 22B. Toutefois, celui-ci peut être disposé sur l'aiguille légèrement en avant de son extrémité arrière, tout en exerçant correctement sa fonction de guidage de l'extrémité arrière de l'aiguille.

Par ailleurs, dans le cas d'une seringue dans laquelle le porte-aiguille 18 est muni de moyens d'étanchéité entre la paroi 20 et les jambages 40, le piston intermédiaire 94 est supprimé. Toutefois, l'organe 66 est avantageusement maintenu afin d'assurer, dans la position analogue à celle de la figure 4, une perforation de la paroi transversale 90 du piston arrière rigoureusement suivant l'axe X-X de la seringue, garantissant ainsi une pénétration axiale correcte de l'aiguille dans le logement 82.

En variante non représentée, les éléments en saillie formés par les bombages 44 et l'élément complémentaire en creux associé formé par le canal annulaire 102 sont inversés. Ainsi, les jambages comportent extérieurement des creux alors que le corps de seringue comporte des saillies complémentaires en vue du maintien du protecteur dans sa position active de protection.

Dans cette variante également, l'organe 66 assure une retenue positive de l'engagement des reliefs associés en saillie et en creux lorsque le protecteur d'aiguille est dans sa position active de protection.

Suivant encore une autre variante représentée sur la figure 8, le piston arrière 14 comporte, en avant de la pastille 80 d'appui du pouce de l'opérateur, des saillies d'encliquetage 150 adaptées pour coopérer avec un épaulement annulaire interne 152 ménagé à l'extrémité arrière du corps de seringue 12 et porté par exemple par l'organe de préhension 25. Cet épaulement délimite un front radial 152A orienté vers l'avant de la seringue prolongé par une rampe 152B dirigée vers l'arrière de la seringue.

Ces saillies d'encliquetage 150 ont par exemple une forme triangulaire, dont la face 154 disposée en avant, en considérant le sens d'enfoncement, constitue une rampe, et dont la face arrière 156 forme un épaulement adapté pour venir en appui sur l'épaulement périphérique intérieur 152 du corps, après dépassement de celui-ci, comme représenté en trait mixte sur la figure 8.

On conçoit que, après enfoncement complet du piston arrière dans le corps de seringue, les saillies d'encliquetage 150 s'enclenchent élastiquement en avant de l'épaulement périphérique 152. Elles assurent ainsi une retenue du piston arrière à l'intérieur du corps de seringue. De ce fait, elles assurent par l'intermédiaire du piston intermédiaire 96 une retenue positive des extrémités arrières du protecteur d'aiguille dans sa position active de protection.

De plus, les saillies d'encliquetage, ainsi que l'épaulement interne ont avantageusement des profils adaptés pour permettre l'émission d'un signal sonore lors du franchissement de l'épaulement périphérique par les saillies d'encliquetage. A cet effet, les saillies d'encliquetage comportent par exemple, comme représenté sur la figure 9, une lame élastique 160 qui est comprimée lors du franchissement de l'épaulement périphérique 152 et qui est libérée après franchissement de celui-ci.

L'existence du signal sonore permet à l'utilisateur de vérifier que le piston arrière d'actionnement est suffisamment enfoncé dans le corps de seringue afin que le protecteur d'aiguille soit réellement en avant de l'extrémité d'injection de l'aiguille.

## Revendications

1. Seringue d'injection (10) comportant, d'une part un corps de seringue allongé (12) comprenant un tube (16) et une paroi avant (20) munie d'une aiguille d'injection (22), et d'autre part un piston arrière d'actionnement (14) monté déplaçable dans le tube (16), laquelle seringue comporte en outre un protecteur mobile (36) de l'extrémité (22A) d'injection de l'aiguille, déplaçable entre une position escamotée dans le corps de seringue en retrait de l'extrémité d'injection (22A) de l'aiguille, et une position active de protection dans laquelle l'extrémité avant du protecteur (36) se trouve en avant de l'extrémité d'injection (22A) de l'aiguille, le protecteur (36) et le corps de seringue (12) comportant des reliefs associés (44, 102) en saillie et en creux de maintien du protecteur (36) dans sa position active de protection, **caractérisée en ce qu'**elle comporte, à l'intérieur du corps de seringue (12), un organe (66) de retenue positive de l'engagement desdits reliefs associés (44, 102) en saillie et en creux lorsque le protecteur d'aiguille (36) est dans sa position active de protection, et **en ce que** le protecteur (36) comporte au moins une surface de came (52) propre à coopérer avec au moins une contre-came (70) portée par l'organe de retenue positive (66) pour provoquer l'engagement desdits reliefs associés (44, 102) en saillie et en creux lors de la phase ultime du déplacement du protecteur (36), sous l'action de l'enfoncement du piston arrière d'actionnement (14).

2. Seringue selon la revendication 1, **caractérisée en ce que** le protecteur (36) comporte des jambages (40) traversant le corps de seringue (12) et dont l'extrémité libre s'étend dans le corps de seringue, les reliefs associés (40, 102) en saillie et en creux étant portés par les extrémités libres des jambages (40) et par le corps de seringue (12), l'organe de retenue positive (66) comportant des moyens (70) d'écartement positif des extrémités libres des jambages (40) vers le corps de seringue (12).

3. Seringue selon les revendications 1 et 2 prises ensemble, **caractérisée en ce que** l'organe de retenue positive (66) est disposé axialement dans le corps de seringue (12), entre les jambages du protecteur d'aiguille, et lesdits moyens d'écartement positif sont formés par une collerette (70) portée par ledit organe de retenue positive (66), laquelle collerette (70) est adaptée pour coopérer, lorsque le protecteur (36) est dans sa position active de protection avec des saillies (50) portées intérieurement par les extrémités libres des jambages (40), lesdites saillies (50) prolongeant chacune une surface de came (52) portée par un jambage (40) avec laquelle coopère ladite collerette (70) formant contre-came lors de la phase ultime de déplacement du protecteur.

4. Seringue selon la revendication 3, **caractérisée en ce que** ladite collerette (70) porte une gorge (71) de réception desdites saillies (50).

5. Seringue selon la revendication 2, 3 ou 4, **caractérisée en ce que** les jambages (40) comportent des moyens (46) d'entraînement de l'organe de retenue positive (66) lors du déplacement du protecteur (36) depuis sa position escamotée vers sa position active de protection et le corps de seringue (12) comporte une butée (20, 32) d'arrêt de l'organe de retenue positive (66) avant l'achèvement du déplacement du protecteur (36) jusqu'à sa position active de protection.

6. Seringue selon la revendication 5 prise avec la revendication 3 ou 4, **caractérisée en ce que** lesdits moyens d'entraînement de l'organe de retenue positive (66) comportent un lamage (46) ménagé dans la surface intérieure des jambages (40) dans lequel est reçue ladite collerette (70), lequel lamage (46) est délimité à l'arrière sur chaque jambage (40) par une surface de came (52).

7. Seringue selon l'une quelconque des revendications précédentes, **caractérisée en ce que** le piston arrière (14) et le corps de seringue (12) comportent des moyens complémentaires adaptés pour produire un signal sonore, lorsque l'enfoncement du piston (14) est suffisant afin que le protecteur d'aiguille soit dans sa position active de protection.

## Patentansprüche

1. Injektionsspritze (10) mit einerseits einem langgestreckten Spritzenkörper (12), der ein Rohr (16) und eine Stirnwand (20) umfasst, die mit einer Injektionsnadel (22) versehen ist, und andererseits einen rückseitigen Betätigungskolben (14) umfasst, der verschiebbar im Rohr (16) angebracht ist, welche Spritze weiterhin einen beweglichen Schutz (26) des Injektionsendes (22A) der Nadel umfasst, der zwischen einer in den Spritzenkörper hinter das Injektionsende (22A) der Nadel eingezogenen Position und einer aktiven Schutzposition versetzbar ist, in der das vordere Ende des Schutzes (36) sich vor dem Injektionsende (22A) der Nadel befindet, wobei der Schutz (36) und der Spritzenkörper (12) zugehörige vorstehende und vertiefte Oberflächenunebenheiten (44, 102) haben, um den Schutz (36) in seiner aktiven Schutzposition zu halten, **dadurch gekennzeichnet, dass** sie im Inneren des Spritzenkörpers (12) ein Element (66) umfasst, das dazu dient, die zugehörigen vorstehenden und vertieften Oberflächenunebenheiten (44, 102) in einer festen Ineingriffnahme zu halten, wenn sich der Schutz (36) der Nadel in der aktiven Schutzposition befindet, und dass der Schutz (36) wenigstens eine Nockenfläche (52) aufweist, die so ausgebildet ist, dass sie mit wenigstens einem Gegennocken (70) zusammenarbeitet, den das Element (66) zum festen Halten trägt, um die Ineingriffnahme der zugehörigen vorstehenden und vertieften Oberflächenunebenheiten (44, 102) bei der letzten Phase der Versetzung des Schutzes (36) unter der Wirkung des Hereindrückens des rückseitigen Betätigungskolbens (14) zu bewirken.

2. Spritze nach Anspruch 1, **dadurch gekennzeichnet, dass** der Schutz (36) Füße (40) umfasst, die durch den Spritzenkörper (12) hindurchgehen und deren freies Endes sich in den Spritzenkörper erstreckt, wobei die zugehörigen vorstehenden und vertieften Oberflächenunebenheiten (40, 102) von den freien Enden der Füße (40) und vom Spritzenkörper (12) gehalten sind, und das Element (66) zum festen Halten Einrichtungen (70) umfasst, die die freien Ende der Füße (40) in Richtung auf den Spritzenkörper (12) sicher im Abstand voneinander halten.

3. Spritze nach Anspruch 1 und 2, **dadurch gekennzeichnet, dass** das Element (66) zum festen Halten axial im Spritzenkörper (12) zwischen den Füßen des Nadelschutzes angeordnet ist und dass die Einrichtungen zum sicheren Halten im Abstand aus einem Kragen (70) bestehen, der von dem besagten Element (66) zum festen Halten gehalten ist, welcher Kragen (70) dann, wenn sich der Schutz (36) in seiner aktiven Schutzposition befindet, mit Vorsprüngen (50) zusammenarbeitet, die im Inneren der freien Ende der Füße (40) gehalten sind, welche Vorsprünge (50) jeweils eine Nockenfläche (52) verlängern, die ein Fuß (40) trägt, mit dem der besagte Kragen (70) zusammenarbeitet, so dass ein Gegennocken während der letzten Phase der Versetzung des Schutzes gebildet ist.

4. Spritze nach Anspruch 3, **dadurch gekennzeichnet, dass** der Kragen (70) eine Kehle (71) zur Aufnahme der Vorsprünge (50) aufweist.

5. Spritze nach Anspruch 2, 3 oder 4, **dadurch gekennzeichnet, dass** die Füße (40) Einrichtungen (46) zum Mitnehmen des Elementes (66) zum festen Halten während der Versetzung des Schutzes (36) aus seiner zurückgezogenen Position in seine aktive Schutzposition umfassen und dass der Spritzenkörper (12) einen Anschlag (20, 32) zum Arritieren des Elementes (66) zum festen Halten vor der Beendigung der Versetzung des Schutzes (36) bis zur aktiven Schutzposition umfasst.

6. Spritze nach Anspruch 5 in Verbindung mit Anspruch 3 oder 4, **dadurch gekennzeichnet, dass** die Einrichtungen zum Mitnehmen des Elementes (66) zum festen Halten eine Senke (46) umfassen, die in der Innenfläche der Füße (40) ausgespart ist, in der der Kragen (70) aufgenommen ist, welche Senke (46) auf der Rückseite an jedem Fuß (40) von einer Nockenfläche (52) begrenzt ist.

7. Spritze nach einem der vorhergehenden Ansprüche, **dadurch gekennzeichnet, dass** der rückseitige Kolben (14) und der Spritzenkörper (12) komplementäre Einrichtungen umfassen, die ein akustisches Signal erzeugen können, wenn das Einschieben des Kolbens (14) ausreicht, damit der Schutz der Nadel sich in der aktiven Schutzposition befindet.

## Claims

1. Injection syringe (10) comprising, firstly, an elongated syringe body (12) comprising a tube (16) and a front wall (20) fitted with an injection needle (22), and, secondly, a rear actuating piston (14) movably mounted in the tube (16), the said syringe further comprises a movable protector (36) for the injecting end (22A) of the needle, movable between a retracted position in the syringe body when the injecting end (22A) of the needle is being withdrawn, and an active protecting position in which the front end of the protector (36) is in front of the injecting end (22A) of the needle, the protector (36) and the syringe body (12) comprising associated projecting and recessed protuberances (44, 102) for maintaining the protector (36) in its active protecting position, **characterised in that** it comprises, inside the syringe body (12), a member (66) for positively retaining the engagement of the said associated projecting and recessed protuberances (44, 102) when the needle protector (36) is in its active protecting position, and **in that** the protector (36) comprises at least one cam surface (52) suitable for co-operating with at least one cam follower (70) carried by the positive retaining member (66) to cause the engagement of the said associated projecting and recessed protuberances (44, 102) during the final displacement phase of the protector (36) under the driving action of the rear actuating piston (14).

2. Syringe according to claim 1, **characterised in that** the protector (36) comprises posts (40) passing through the syringe body (12) and its free end extends into the syringe body, the associated projecting and recessed protuberances (44, 102) being carried by the free ends of the posts (40) and by the syringe body (12), the positive retaining member (66) comprising means (70) for positively separating the free ends of the posts (40) towards the syringe body (12).

3. Syringe according to claims 1 and 2 together, **characterised in that** the positive retaining member (66) is axially arranged in the syringe body (12), between the posts of the needle protector, and the said positive separating means are formed by a flange (70) carried by the said positive retaining member (66), the said flange (70) is adapted to co-operate, when the protector (36) is in its active protecting position, with the projections (50) carried internally by the free ends of the posts (40), the said projections (50) each extending on a cam surface (52) supported by a post (40) with which the said flange (70) co-operates to form a cam follower during the final displacement phase of the protector.

4. Syringe according to claim 3, **characterised in that** the said flange (70) has a groove (71) for receiving the said projections (50).

5. Syringe according to claim 2, 3 or 4, **characterised in that** the posts (40) comprise means (46) for driving the positive retaining member (66) when the protector (36) is moved from its retracted position to its active protecting position and the syringe body (12) comprises means (20, 32) for stopping for the positive retaining member (66) before the protector (36) has been moved to its active protecting position.

6. Syringe according to claim 5 together with claim 4 or 5, **characterised in that** the said drive means for the positive retaining member (66) comprise a facing (46) arranged in the internal surface of the posts (40) in which the said flange (70) is received, the said facing (46) is defined at the back of each post (40) by a cam surface (52).

7. Syringe according any of the preceding claims, **characterised in that** the rear piston (14) and the syringe body (12) comprise complementary means adapted to produce an audio signal when the piston (14) has been driven sufficiently to put the needle protector into its active protecting position.
